# EUROPEAN PATENT APPLICATION

(11) **EP 0 569 600 A1**
(43) Date of publication of application: **18.11.1993**
(21) Application number: 92924022.4
(22) Date of filing: 27.11.1992
(51) Int. Cl.: H04L 12/00, H04L 12/40

(54) **METHOD FOR RECOVERING FAILURED TRANSMISSION LINE**

(30) Priority: 02.12.1991 JP 344009/91
(71) Applicant: THE FURUKAWA ELECTRIC CO., LTD., Tokyo 100 (JP)
(72) Inventor: AKIMOTO, Mitsuru The Furukawa Electric Co., Ltd., Tokyo 100 (JP); TANAKA, Motoharu The Furukawa Electric Co., Ltd., Tokyo 100 (JP); HASHIMOTO, Kyosuke The Furukawa Electric Co.,, Tokyo 100 (JP)
(74) Representative: Modiano, Guido, Dr.-Ing.
(86) International application number: JP9201556
(87) International publication number: WO9311620

(57) **Abstract**

A method for removing failures in which multinodes (10, 11, 12) are connected to common bus lines (A, B, C), and one of the multinodes, a failure detecting node (12), detects the abnormality of the the multinodes and the bus lines, and using the bus line (C) as a controlling line, the node (12) sends out a potential through the controlling line (C) to change the statuses of the bus lines. In the method, when the failure detecting node (12) detects a communication abnormality, it examines the bus connection current status (transmission mode), drives a controlling circuit (23) according to the kind of the detected communication abnormality and the detected transmission mode, and changes the status of the controlling line (C), thereby to make the nodes (12) change the bus lines.

## Description

### Technical Field

This invention relates to a multiplex transmission system for transmitting data among multiplex nodes interconnected by three or more signal transmission lines, and more particularly, to a control method for multiplex nodes applied in the case of fault of a signal transmission line in a multiplex transmission system.

### Background Art

Multiplex transmission systems of this type include a LAN (local area network)-based transmission system, and a typical example of LAN is an automobile multiplex transmission system. Automobile multiplex transmission systems must be high in reliability, and are required to avoid situations where data transmission is entirely disabled due to fault of signal transmission lines (hereinafter called "buses"). To prevent the data transmission incapability, when a bus fails, the bus is isolated from the system and data is transmitted via the other buses. A conventional method for carrying out such technique is disclosed in International Publication No. WO90/01739 (Unexamined Japanese Patent Publication No. 3-500477). This publication describes a method wherein each multiplex node monitors bus waveforms, it is concluded that fault of a bus has occurred if the bus fails to transmit data for a predetermined period of time, and buses on which signals are detectable are selected for use; and a method wherein the potentials of buses are monitored, it is concluded that a bus has failed if the potential of the bus becomes outside a predetermined range, and buses on which signals are detectable are selected for use.

In Japanese Patent Application No. 2-113751 is disclosed a three-bus transmission system including three buses connected to a plurality of multiplex nodes, and in which a predetermined one of the buses functions as a control line. This publication proposes a method wherein, when a bus fails, a fault detecting node, which is a predetermined one of the multiplex nodes, provides a bus switch command to the other multiplex nodes. In this method, the fault detecting node sets the potential of the control line to an intermediate potential, power-supply potential, or ground potential, so that the other multiplex nodes can detect the set potential for the selection of buses.

In the fault control methods disclosed in Unexamined Japanese Patent Publication No. 3-500477, each multiplex node detects signals on the buses, or the potentials of the buses or data absence time is monitored, and when an abnormal state is detected, the bus selection is controlled. Thus, the methods are disadvantageous in that each multiplex node requires a window comparator or a counter circuit and is increased in software load.

In the fault control method disclosed in Japanese Patent Application No. 2-113751, the buses are switched after the fault detecting node detects a communication error. Thus, the fault detecting node alone requires a fault detecting circuit and is increased in software load, and the method is advantageous over the aforementioned methods in that the system can be simplified and the fault control is centralized. In this method, however, a plurality of buses are checked for fault in predetermined order. Accordingly, if a bus which is checked later in the fault detection fails, the processing time is prolonged compared with the case of fault of a bus which is checked earlier in the fault detection, thus increasing the load on software. Further, in this method, when the buses are selected based on the potential of the control line of the three-bus transmission system, two switch means for setting the potential of the control line can operate simultaneously, causing a short circuit between the power supply and the ground, with the result that data transmission becomes unfeasible.

### Disclosure of the Invention

One object of this invention is to provide a transmission line fault control method which reduces software load on a fault detecting node for controlling the selection of buses, and which shortens the time required from the occurrence of fault of a bus or multiplex node to the start of selection control executed by the fault detecting node.

Another object of this invention is to provide a transmission line fault control method which prevents simultaneous closing of switch means for setting the potential of a control line in whatever state a computer of the fault detecting node is.

To achieve the above objects, according to a fault control method of this invention, when a communication error such as a transmission error or a reduction in the number of nodes in a transmission system is detected in data transmission which is carried out among the nodes as needed, a fault detecting node detects the present state of bus connection (transmission mode), and sets a potential based on the detected communication error and the detected transmission mode, to change the states of signal transmission lines. Further, the fault control method according to this invention provides a logic whereby two switch means are prevented from being closed at the same time even when a computer for carrying out the fault control or gates for controlling the switch means fail.

As mentioned above, according to this invention, the fault state is identified and the buses are selected based on the detected conditions, and the bus selection of the individual nodes is effected instantly via the control line. Since the buses are selected within a short control time, the buses can be switched at high speed, and thus the control method of this invention can be suitably applied to high-speed signal transmission.

### Brief Description of the Drawings

The drawings illustrate one embodiment of this invention, wherein:
FIG. 1 is a block diagram showing the configuration of a multiplex transmission system for carrying out a transmission line fault control method according to this invention;
FIG. 2 is a logic circuit diagram of a control circuit shown in FIG. 1;
FIG. 3 is a chart illustrating bus selection modes used in this invention;
FIG. 4 is a flowchart showing a fault control process executed by a fault detecting node shown in FIG. 1;
FIG. 5 is a flowchart showing the fault control process executed by the fault detecting node;
FIG. 6 is a flowchart showing the fault control process executed by the fault detecting node; and
FIG. 7 is a diagram showing ACK bits which are necessary for detecting an abnormal reduction in the number of nodes.

### Best Mode of Carrying Out the Invention

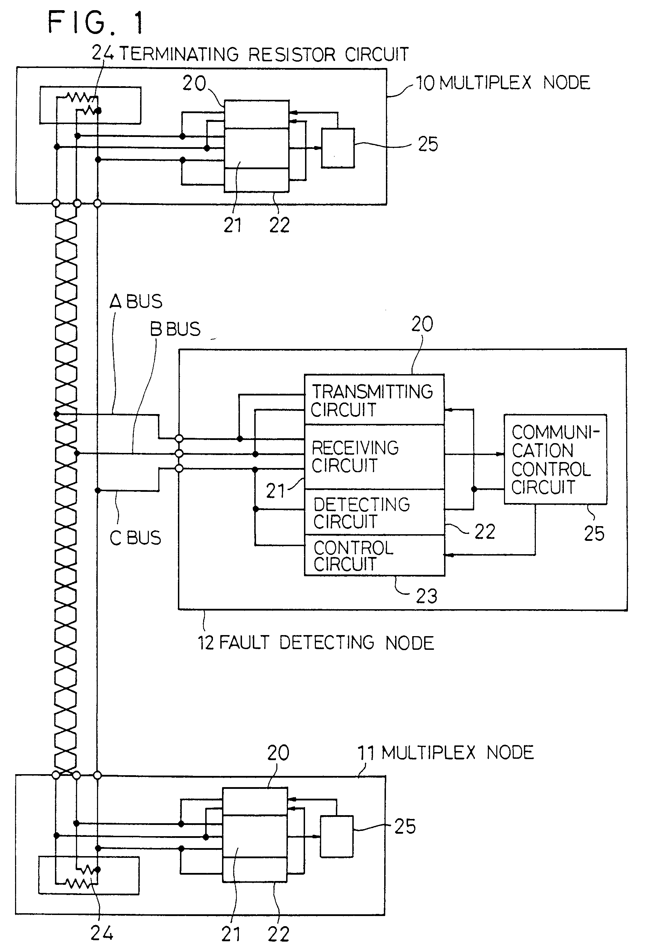

An embodiment of this invention will be described with reference to FIGS. 1 through 7.

Referring first to FIG. 1, a plurality of multiplex nodes 10 and 11 and a fault detecting node 12 are individually connected in parallel to three buses A, B and C, for transferring data signals among the nodes.

The multiplex nodes 10 and 11 each comprise a transmitting circuit 20 for transmitting data signals to the buses, a receiving circuit 21 for detecting the potential difference between the buses, a detecting circuit 22 for detecting the voltage of a control line which is a specific one of the buses, e.g., the bus C, and for supplying the detected voltage to a communication control circuit 25 etc., a terminating resistor circuit 24 connected to ends of the buses A, B and C, and the communication control circuit 25 including a communication chip and a CPU. A shielded cable may be used for the buses to reduce unwanted radiation noise and prevent external noise, and in this case, the shielded cable may be used as the control line (bus C), thereby reducing the cost of the buses.

The communication control circuit 25 selects a transmission mode (described later) for selecting the buses to be used, in accordance with the voltage of the control line C detected by the detecting circuit 22.

The fault detecting node 12 comprises a transmitting circuit 20, a receiving circuit 21, a detecting circuit 22 and a communication control circuit 25 having functions similar to those of the corresponding circuits in the multiplex nodes 10 and 11, and further comprises a control circuit 23 for changing the voltage of the control line C to a predetermined potential.

Table 1 below shows the relationship of faults of the buses, communication errors detected (transmission error or abnormal reduction in the number of nodes), potential of the bus C in the case of fault, and transmission mode selected after fault control, observed when a fault control method according to this invention is applied to the multiplex transmission system shown in FIG. 1.

**TABLE 1**

| Fault of buses | Communication error detected | Potential of bus C | Transmission mode |
|---|---|---|---|
| Open circuit of bus A | No reduction of nodes, and/or No communication error | V1 | Mode 1 |
| Open circuit of bus B | No reduction of nodes, and/or No communication error | V1 | Mode 1 → Mode 2 |
| Short circuit of bus A to power supply | No transmission error, and/or No communication error | V2 | Mode 1 |
| Short circuit of bus B to power supply | No transmission error, and/or No communication error | V2 | Mode 2 |
| Short circuit of bus A to ground | No transmission error, and/or No communication error | V3 | Mode 1 |
| Short circuit of bus B to ground | No transmission error, and/or No communication error | V3 | Mode 2 |
| Short circuit between buses | Transmission error | V1 | Mode 1 |
| Open circuit of buses A and B | Reduction of nodes | V1 | Mode 1 → Mode 2 → Mode 0 |

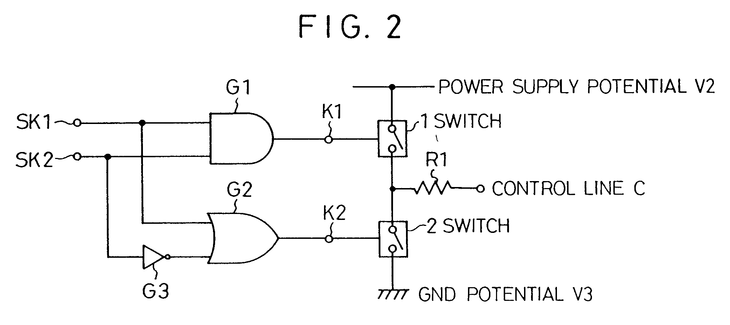

FIG. 2 is a logic circuit diagram of the control circuit 23 which serves as a switching control means for setting the potential of the control line according to this invention, and Table 2 shows the relationship of the states of input signals (SK1, SK2) shown in FIG. 2 and transmission modes.

**TABLE 2**

| SK1 | SK2 | K1 | K2 | Switch 1 | Switch 2 | Potential of bus C | Transmission mode |
|---|---|---|---|---|---|---|---|
| 0 | 0 | 0 | 1 | Off | Off | V1 | Mode 0 |
| 1 | 0 | 0 | 1 | Off | Off | V1 | Mode 0 |
| 0 | 1 | 0 | 0 | Off | On | V3 | Mode 2 |
| 1 | 1 | 1 | 1 | On | Off | V2 | Mode 1 |

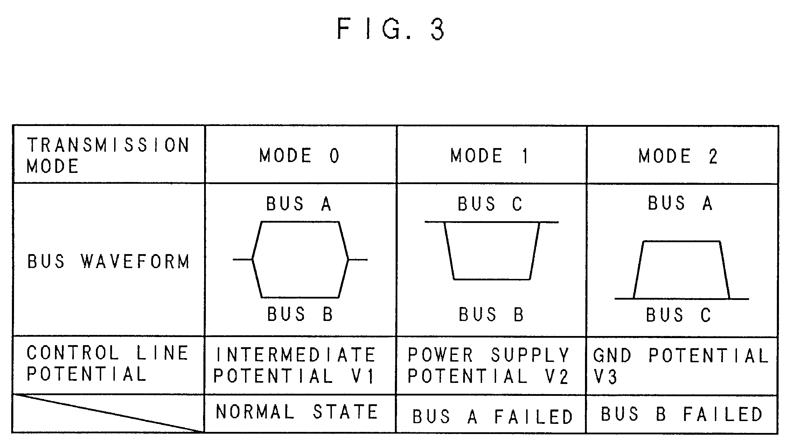

Referring to FIG. 2, the signals SK1 and SK2 (output from a computer of the fault detecting node) controls logic circuits G1 (AND gate), G2 (OR gate) and G3 (NOT gate), and signals K1 and K2 are output. The output signals K1 and K2 control switches 1 and 2 serving as switching means. The switch 1 is closed when the output signal K1 of the logic circuit G1 is high, and the switch 2 is closed when the output signal K2 of the logic circuit G2 is low. Namely, the open/close operation of the switches 1 and 2 of the control circuit 23 is controlled to set the potential of the control line C to an intermediate potential V1, power supply potential V2 or ground potential V3 through a resistor R1, as shown in FIG. 3, so that each multiplex node can detect the set potential to select the buses in accordance with the transmission mode. As shown in FIG. 3, in a normal state wherein the potential of the control line C is set to the intermediate potential V1, the transmission mode is set to mode 0 and the buses A and B are selected. In the case of a fault of the bus A wherein the power supply potential V2 is set, mode 1 is set as the transmission mode and the buses B and C are selected; in the case of a fault of the bus B wherein the ground potential V3 is set, mode 2 is set as the transmission mode and the buses A and C are selected.

The use of the logic circuits makes it possible to prevent the switches 1 and 2 from being closed simultaneously, as shown in Table 2, in whatever state the signals SK1 and SK2 are, that is, in whatever state the computer for producing the signals SK1 and SK2 is. Thus, in this embodiment, short circuit between the power supply and the ground can be prevented.

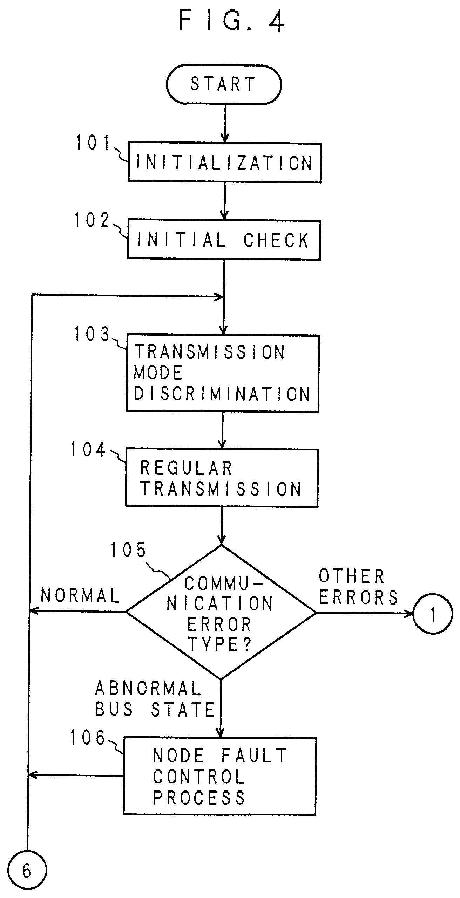

A bus fault control process executed by the fault detecting node will be now described with reference to the flowchart of FIGS. 4 to 6. The fault control process illustrated in the flowchart is executed with respect to the three-bus transmission system shown in FIG. 1.

Normal communication is ensured by the basic operation of the fault detecting node 12; namely, the communication control circuit 25 detects a communication error by determining whether transmission frames on the buses are normal, and selects the transmission mode (that is, the control circuit 23 changes the potential of the control line C under the control of the communication control circuit 25). Further, in this embodiment, the communication control circuit 25 discriminates between types of communication errors, to optimize the fault control and shorten the fault control time.

Referring to FIG. 4, the fault detecting node 12 first carries out initialization (Step 101) and then initial check (Step 102). Step 102 is a fault detection step, wherein the fault detecting node 12 changes the potential of the bus (control line) C from V1 (transmission mode 0) to V2 (transmission mode 1) and then to V3 (transmission mode 2), as shown in FIG. 3, and carries out transmission in the individual transmission modes.

The subsequent Step 103 is a transmission mode detection step, wherein the fault detecting node 12 executes a warning process (warning notification) if the detected transmission mode is mode 1 or 2 even though the transmission mode has been set to mode 0 and no communication error is occurring.

In the next Step 104, the fault detecting node 12 carries out regular data transmission, and in Step 105, detects a communication error and the type thereof. While the communication is normal, Steps 103, 104 and 105 are repeatedly executed. If a communication error is detected and if the detected error is identified as a bus abnormality (data remains on the buses), it is concluded that a node has failed, and a node fault control process (Step 106) is executed. The bus abnormality represents a state in which data is continuously transmitted to the buses, which state can be regarded as a fault of a multiplex node.

In the node fault control process, the fault detecting node 12 changes the transmission mode and carries out communication with the other nodes, thus establishing a transmission mode which permits communication with at least one node.

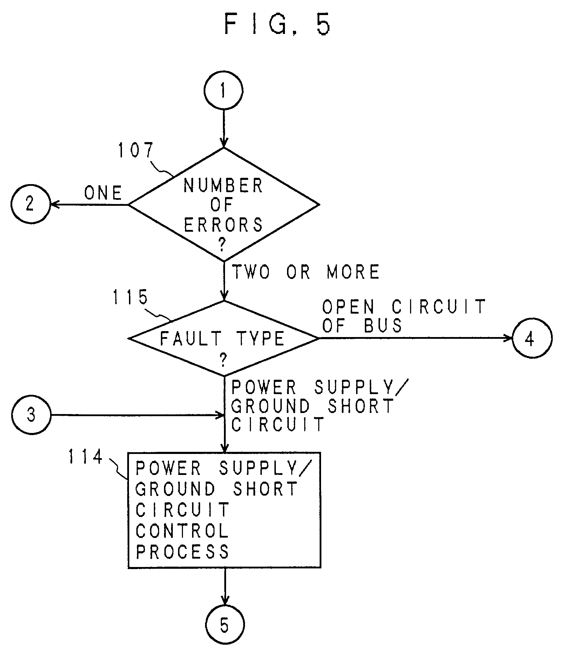

If some other type of communication error is detected in Step 105, the flow proceeds to Step 107 (see FIG. 5). Other types of communication errors include a transmission error (transmission incapability) and an abnormal reduction in the number of nodes (reduction of the nodes capable of communication). The following is a description of these communication errors.

The transmission error represents an abnormal state in which transmission data fails to be transmitted to the buses, that is, a sending node is unable to receive the data transmitted therefrom. The abnormal reduction in the number of nodes represents an abnormal state wherein a predetermined number of ACK bits do not exist on the buses.

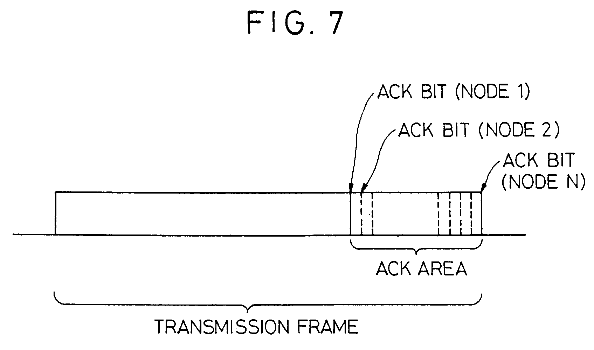

The ACK bits are the signals which the receiving nodes send upon appropriately receiving a transmission frame on the buses. As shown in FIG. 7, the ACK bits are sent to a specific area (ACK area) in the transmission frame on the buses, i.e., to respective bit areas assigned to the multiplex nodes.

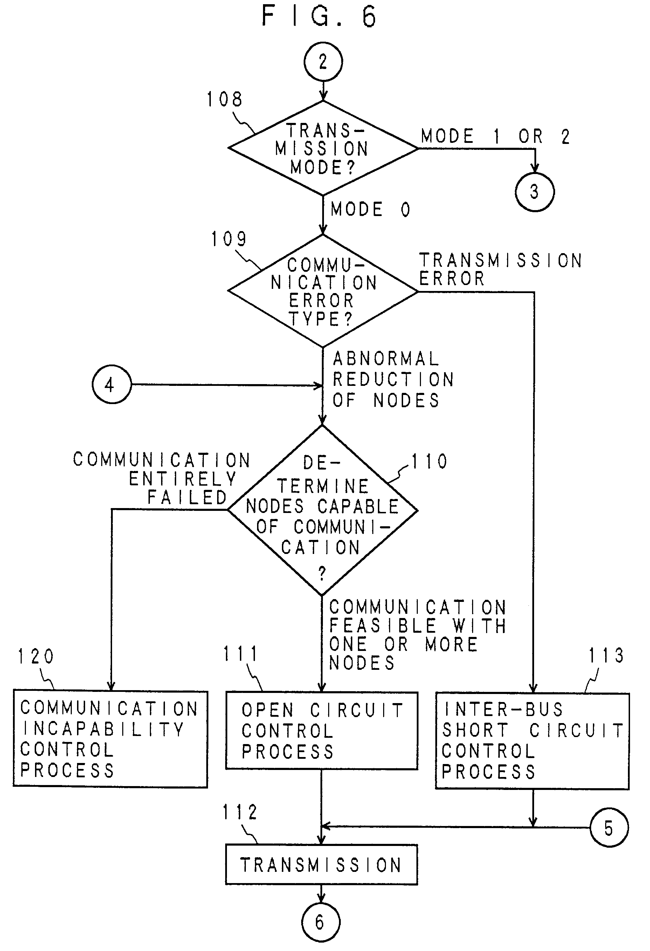

Referring now to FIG. 5, the fault detecting node 12 determines the number of occurrences of the communication error, or more specifically, determines whether the communication error is detected for the first time or was detected once before (Step 107). FIG. 6 illustrates a fault control process (Steps 108 to 114) which is executed when the communication error is detected for the first time, wherein fault type is discriminated and the fault control is carried out accordingly. In a fault control process (Step S115) which is executed when the error was detected before, fault control is carried out based on the fault state which was determined when the error was detected for the first time, and the number of occurrences of the error. Thus, in this embodiment, load of the fault control process can be reduced where the error is detected a plurality of times.

The fault control process executed when error is detected for the first time will be described with reference to FIG. 6.

In Step 108, the transmission mode is detected. Specifically, if the detected transmission mode is a mode other than mode 0 (the potential of the bus C is not V1) even though the fault detecting node 12 has set the transmission mode to mode 0, then the fault detecting node 12 judges that a bus is short-circuited to the power supply or the ground, and executes a power supply/ground short circuit control process (Step 114 described later). If the detected transmission mode is mode 0, it is concluded that open circuit of a bus or short circuit between the buses has occurred, and the flow proceeds to the subsequent step for fault determination (Step 109).

In Step 109, the fault detecting node 12 discriminates the type of communication error, and further determines whether the error is a transmission error or an abnormal reduction in the number of nodes. Specifically, in the case of a transmission error in which no waveform appears on the buses at the time of transmission, it is concluded that short circuit between the buses has occurred, and an inter-bus short circuit control process (Step 113 described later) is carried out. When the detected communication error is identified as an abnormal reduction in the number of nodes, it is concluded that open circuit of a bus has occurred, and it is determined which nodes are capable of communication (Step 110 described later). If at least one of the nodes is capable of communication, a bus disconnection control process (Step 111 described later) is executed.

In Step 110, the fault detecting node 12 determines which nodes are capable of communication. If the node 12 is unable to establish communication with any of the nodes, then the transmission mode is reset to mode 0, and the flow proceeds to a communication incapability control process (Step 120). In Step 120, a process such as a warning process is executed. If it is concluded in Step 110 that some other node is capable of communication, the node 12 judges that open circuit of a bus has occurred, and the bus open circuit control process (Step 111) is carried out.

In the case where the communication error is detected for the first time, the control circuit 23 sets the potential of the bus C to V2 (the transmission mode is set to mode 1) (Step 111), and carries out transmission by means of the transmitting circuit 20 (Step 112). In the transmission mode 1, transmission is carried out using the buses B and C, as shown in FIG. 3, and thus open circuit of the bus A can be coped with by the fault control process. In the case where the communication error was detected once before, the control circuit 23 sets the potential of the bus C to V3 (the transmission mode is set to mode 2) (Step 111), and transmission is carried out by the transmitting circuit 20 (Step 112). In the transmission mode 2, transmission is carried out by means of the buses A and C, as shown in FIG. 3, and accordingly, disconnection of the bus B can be coped with by the fault control process. In the case where the communication error was detected twice before, it is concluded that the buses A and B are both open circuit, the control circuit 23 sets the potential of the bus C to V1 (the transmission mode is set to mode 0) (Step 111), and transmission is carried out by the transmitting circuit 20 (Step 112). When the buses A and B are both open circuit, the fault detecting node 12 is unable to carry out communication with each node in any transmission mode (though communication with at least one node is possible), and in this case, the fault control process is not carried out even if an abnormal reduction of nodes occurs thereafter.

Step 113 is a step for the inter-bus short circuit control process, wherein the control circuit 23 sets the potential of the bus C to V2 (the transmission mode is set to mode 1), and transmission is carried out by means of the transmitting circuit 20 (Step 112).

Step 114 is the power supply/ground short circuit control process, and in the case where the communication error is detected for the first time, the control circuit 23 sets the potential of the bus C in accordance with the transmission mode automatically selected. Specifically, if the transmission mode is in accord with V2 though the fault detecting node 12 has set the potential of the bus C to V1 (the transmission mode is set in mode 0), the control circuit 23 sets the potential of the bus C to V2. In the case of the transmission mode being in accord with V3, the potential of the bus C is set to V3. Where the communication error was detected once before, the control circuit 23 sets the potential of the bus C to the opposite potential (i.e., V3 is set if the potential set when the communication error was detected for the first time is V2, and vice versa), and transmission is carried out by the transmitting circuit 20 (Step 112).

Even if any one of the gates G1 to G3 fails, the switches 1 and 2 are prevented from being closed at the same time as long as the communication control circuit 25 controls the switches 1 and 2 in accordance with the flowchart of FIGS. 4 to 6. For example, even when the switch 1 is closed due to a short mode fault of the gate G1, the switch 2 is not closed because the flow proceeds to the same steps as in the case of a short circuit of the bus A to the power supply. Similarly, when the gate G2 or G3 develops a short mode fault, the switch 1 is not closed since the flow proceeds to the same steps as in the case of a short circuit of the bus B to the ground.

Thus, in the fault control method according to this embodiment, when a communication error is detected by the fault detecting node, which is a specific node among a plurality of multiplex nodes connected in parallel to three buses, the present state of bus connection (transmission mode) and the type of communication error are determined, and based on the determined conditions, the potential of the control line is set such that a specific transmission mode is established, whereby each node can select the buses in accordance with the set potential detected by the receiving circuit. As seen from FIGS. 4 to 6, after a bus fault occurs, the bus selection is, in most cases, accomplished in one control cycle of the fault control process, and thus the buses can be selected and switched at high speed. Accordingly, the fault control method of this embodiment is suited to high-speed signal transmission.

Further, in this embodiment, the power supply-side switch 1 for connecting the control line to the power supply and the ground-side switch 2 for connecting the control line to the ground are prevented from being closed at the same time, and accordingly, a short circuit between the power supply and the ground can be prevented even if the computer or the gates for controlling the switches fail.

Where technical features mentioned in any claim are followed by reference signs, those reference signs have been included for the sole purpose of increasing the intelligibility of the claims and accordingly, such reference signs do not have any limiting effect on the scope of each element identified by way of example by such reference signs.

## Claims

1. A fault control method for three or more signal transmission lines to which a plurality of multiplex nodes are connected, in which at least one multiplex node among said plurality of multiplex nodes detects abnormality of the individual multiplex nodes and/or the individual signal transmission lines, and sets the potential of at least one control line which is one of the signal transmission lines, to change states of the signal transmission lines,
characterized in that, upon detecting a communication error of data transmission which is carried out among the multiplex nodes as needed, said at least one multiplex node detects the states of the presently used signal transmission lines, and changes the state of the control line by setting the potential thereof based on the detected communication error and the detected states of the signal transmission lines.

2. A fault control method according to claim 1, characterized in that said at least one multiplex node judges that a multiplex node has failed, when at least two signal transmission lines continuously develop a potential difference therebetween.

3. A fault control method according to claim 1, characterized in that said at least one multiplex node determines abnormality of the signal transmission lines based on a transmission error and/or an abnormal reduction of the nodes, for carrying out fault control.

4. A fault control method according to claim 1, characterized in that said at least one multiplex node comprises power supply-side switch means for connecting the control line to a power supply, and ground-side switch means for connecting the control line to ground, and carries out switching control such that the power supply-side switch means and the ground-side switch means are not simultaneously closed.

5. A fault control method according to claim 1, characterized in that said at least one multiplex node comprises power supply-side switch means for connecting the control line to a power supply, ground-side switch means for connecting the control line to ground, and switching control means for preventing the power supply-side switch means and the ground-side switch means from being closed simultaneously, and carries out control such that the power supply-side switch means and the ground-side switch means are not simultaneously closed even when the switching control means fails.

6. A fault control method according to claim 1, characterized in that said at least one multiplex node judges that a multiplex node has failed when at least two signal transmission lines continuously develop a potential difference therebetween, determines abnormality of the signal transmission lines based on a transmission error and/or an abnormal reduction of the nodes, and identifies a fault state by monitoring the potentials of the signal transmission lines then used.

7. A fault control method according to claim 1, characterized in that said signal transmission lines comprise a shielded cable.
